# EUROPÄISCHE PATENTANMELDUNG

(11) **EP 0 615 733 A1**
(43) Veröffentlichungstag der Anmeldung: **21.09.1994**
(21) Anmeldenummer: 94250053.9
(22) Anmeldetag: 02.03.1994
(51) Int. Cl.: A61F 2/30, A61F 2/32, A61L 27/00

(54) **Gelenkersatz mit Kugeln**

(30) Priorität: 15.03.1993 DE 9304038 U
(71) Anmelder: AAP GmbH & Co. Betriebs KG, D-12099 Berlin (DE)
(72) Erfinder: Ahrens, Uwe, D 14167 Berlin (DE)
(74) Vertreter: Meissner, Peter E., Dipl.-Ing.

(57) **Zusammenfassung**

Die Erfindung betrifft ein Gelenk für den Gelenktotalersatz am Knie, der Hüfte, der Schulter oder anderen Gelenken, bestehend aus den beiden Gelenkteilen (2, 3) und einem dazwischen angeordneten die Reibung vermindernden Einsatz.

Erfindungsgemäß besteht der Einsatz aus einem Kugelkäfig mit darin angeordneten Kugeln (6).

## Beschreibung

Die Erfindung betrifft ein Gelenk für den Gelenktotalersatz am Knie, der Hüfte, der Schulter oder anderen Gelenken, bestehend aus den beiden Gelenkteilen sowie einem dazwischen angeordneten, die Reibung vermindernden Einsatz.

Bei den aus der Praxis bekannten Endoprothesensystemen wie z.B. Hüftendoprothesen ist zwischen der Gelenkpfanne und dem darin einsetzbaren Kugelkopf beispielsweise ein Einsatz angeordnet aus einem Kunststoffmaterial. Dieser Einsatz ist quasi wie ein den Kugelkopf umgebender Kugelaufsatz ausgebildet. Es hat sich gezeigt, daß die Gefahr der Lockerung des Implantates gegeben ist, da bei diesen herkömmlichen die Reibung vermindernden Einsätzen ein Abrieb auftritt, der dann zu einer Verringerung der Stärke des Einsatzes führt und damit zu einer Lockerung des gesamten Gelenktotalersatzes. Darüber hinaus können sich Probleme aus dem Abrieb ergeben, der aus der Gelenkpfanne heraustritt und in das Gewebe einwachsen kann. Dies gilt auch für andere Materialien wie Paarungen von metallischen oder keramischen Einsätzen.

Aufgabe der Erfindung ist es daher, ein verbessertes Gelenk für den Gelenktotalersatz zu Verfügung zu stellen.

Gelöst wird diese Aufgabe erfindungsgemäß dadurch, daß der bisher übliche Einsatz durch einen Kugelkäfig erstetzt wird und daß in diesem Kugelkäfig nach einer weiteren Ausbildung der Erfindung Kugeln z.B. Keramikkugeln angeordnet sind.

Ein derartiger Kugelkäfig mit Kugeln aus Keramik ist hohen Beanspruchungen gewachsen ohne daß es zu einem Abrieb kommen kann, so daß damit die oben beschriebenen Nachteile vermieden werden können.

Die Erfindung soll nachfolgend an einer Prinzipskizze eines Hüftgelenkes erläutert werden.

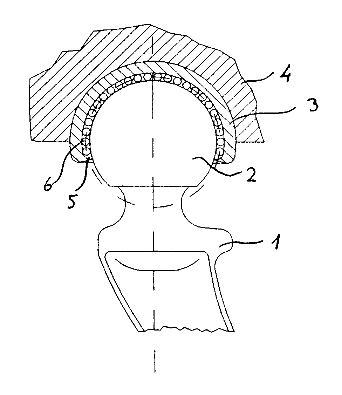

In der Figur ist mit 1 die Hüftendoprothese bezeichnet. Diese schließt mit einem Kugelkopf 2 ab. In die natürliche Gelenkpfannne, in der Figur nur teilweise dargestellt und mit 4 bezeichnet, wird eine Pfanne 3 einzementiert. Zwischen der Pfanne 3 und dem Kugelkopf 2 befindet sich der Kugelkäfig mit den Kugeln 6. Der Spalt zwischen Pfanne 3 und Kugelkopf 2 wird am Abschluß der Pfanne verschlossen, wie mit 5 angedeutet. Hierdurch wird der Eintritt von Gewebe oder dergleichen verhindert.

## Patentansprüche

1. Gelenk für den Gelenktotalersatz am Knie, der Hüfte, der Schulter oder anderen Gelenken, bestehend aus den beiden Gelenkteilen und einem dazwischen angeordneten die Reibung vermindernden Einsatz,
dadurch gekennzeichnet,
daß der Einsatz aus einem Kugelkäfig mit darin angeordneten Kugeln besteht.

2. Gelenk nach Anspruch 1,
dadurch gekennzeichnet,
daß die Kugeln (6) aus Kermaikmaterial bestehen.

3. Gelenk nach einem der Ansprüche 1 oder 2,
dadurch gekennzeichnet, daß der Spalt zwischen den Gelenkteilen am Abschluß des Einsatzes abgekapselt ist.
